(19) Europäisches Patentamt
European Patent Office
Office européen des brevets

(11) EP 4 122 465 A1

(12) EUROPEAN PATENT APPLICATION
published in accordance with Art. 153(4) EPC

(43) Date of publication:
**25.01.2023 Bulletin 2023/04**

(21) Application number: **21787967.5**

(22) Date of filing: **15.04.2021**

(51) International Patent Classification (IPC):
***A61K 31/4545*** *(2006.01)* ***A61K 31/138*** *(2006.01)*
***A61K 31/519*** *(2006.01)* ***A61K 31/565*** *(2006.01)*
***A61K 45/06*** *(2006.01)* ***A61P 15/00*** *(2006.01)*
***A61P 35/00*** *(2006.01)* ***A61P 35/04*** *(2006.01)*
***A61P 43/00*** *(2006.01)*

(52) Cooperative Patent Classification (CPC):
**A61K 31/138; A61K 31/4545; A61K 31/519; A61K 31/565; A61K 45/06; A61P 15/00; A61P 35/00; A61P 35/04; A61P 43/00**

(86) International application number:
**PCT/JP2021/015546**

(87) International publication number:
**WO 2021/210636 (21.10.2021 Gazette 2021/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.04.2020 JP 2020074386**

(71) Applicant: **Eisai R&D Management Co., Ltd
Bunkyo-ku, Tokyo 112-8088 (JP)**

(72) Inventors:
- **NISHIBATA Kyoko
Tsukuba-shi, Ibaraki 300-2635 (JP)**
- **FUKUSHIMA Sayo
Tsukuba-shi, Ibaraki 300-2635 (JP)**
- **KAWANO Satoshi
Tsukuba-shi, Ibaraki 300-2635 (JP)**
- **MIYANO Saori
Tsukuba-shi, Ibaraki 300-2635 (JP)**

(74) Representative: **Hoffmann Eitle
Patent- und Rechtsanwälte PartmbB
Arabellastraße 30
81925 München (DE)**

(54) **BREAST CANCER THERAPEUTIC AGENT**

(57) Provided is a therapeutic agent for treating breast cancer that has developed resistance to administration of CDK4/6 inhibitors and estrogen antagonists, the agent containing 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof.

*Fig.1*

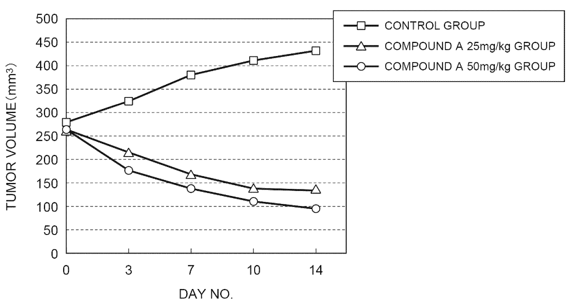




Processed by Luminess, 75001 PARIS (FR)

## Description

## Technical Field

The present invention relates to a breast cancer therapeutic agent that includes a monocyclic pyridine derivative or a pharmacologically acceptable salt thereof, having FGFR inhibiting activity. More specifically, the invention relates to a breast cancer therapeutic agent that includes 5-((2-(4-(1-(2-hydroxyethyl)piperidin-4-yl)benzamide)pyridin-4-yl)oxy)-6-(2-methoxyethox y)-N-methyl-1H-indole-1-carboxamide or a pharmacologically acceptable salt thereof.

## Background Art

[Chemical Formula 1]

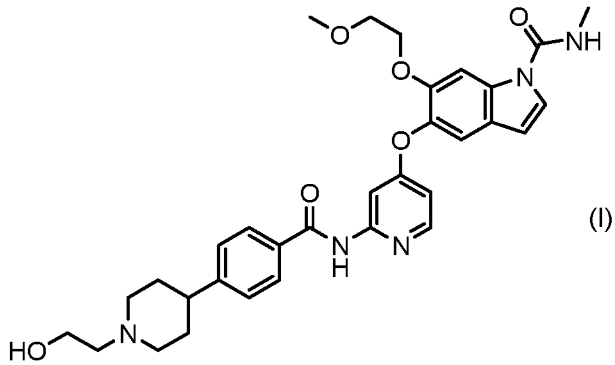

(I)

The compound 5-((2-(4-(1-(2-hydroxyethyl)piperidin-4-yl)benzamide)pyridin-4-yl)oxy)-6-(2-methoxyethox y)-N-methyl-1H-indole-1-carboxamide, represented by formula (I), is known as an inhibitor against fibroblast growth factor receptors (FGFR) 1, 2 and 3, and has been reported to have a cell growth inhibitory effect against stomach cancer, lung cancer, bladder cancer and endometrial cancer (PTL 1). The compound has also been reported to have a high therapeutic effect for bile duct cancer (PTL 2), breast cancer (PTL 3) and hepatocellular carcinoma (PTL 4). Known pharmacologically acceptable salts of the compound include succinic acid salts and maleic acid salts (PTL 5).

Cyclin-dependent kinase 4/6 (CDK4/6) inhibitors such as palbociclib and abemaciclib are used for treatment of estrogen receptor-positive and HER2-negative breast cancer (NPL 1).

Estrogen receptor antagonists such as tamoxifen and fulvestrant are also used for treatment of estrogen receptor-positive breast cancer (NPL 2).

Breast cancer is classified based on expression of estrogen receptor, progesterone receptor and human epidermal growth factor receptor type 2 (HER2), and drug therapy is implemented to match each type, concomitant with extraction surgery at the affected site.

Estrogen receptor-positive and HER2-positive types constitute a large portion of breast cancer in patients. It has been reported that administering palbociclib and fulvestrant to breast cancer patients with these types significantly improves the overall response rate (ORR) compared to administration of fulvestrant alone (NPL 3). However, it has also been reported that continued administration of palbociclib and fulvestrant produces resistance, resulting in loss of effect (NPL 4).

## Citation List

## Patent Literature

[PTL 1] U.S. Patent Application Publication No. 2014-0235614
[PTL2] U.S. Patent Application Publication No. 2018-0015079
[PTL 3] U.S. Patent Application Publication No. 2018-0303 817
[PTL4] International Patent Publication No. WO2019/189241
[PTL 5] U.S. Patent Application Publication No. 2017-0217935

**Non-Patent Literature**

[NPL 1] Bottcher et al., Acta Oncologica, 2019, 58(2), 147-153.
[NPL2] Howell et al., Journal of Clinical Oncology, 2004, 22(9), 1605-1613.
[NPL3] Seki et al., In vivo, 2019, 33, 2037-2044.
[NPL4] Nayar et al., Nature Genetics, 2019, 51, 207-216.

## Summary of Invention

### Technical Problem

It is an object of the present invention to provide a therapeutic agent for breast cancer that has become resistant to administration of CDK4/6 inhibitors and estrogen receptor antagonists.

### Solution to Problem

As a result of conducting much research in light of the current situation, the present inventors have completed this invention upon finding that the compound represented by formula (I) above exhibits a high therapeutic effect against breast cancer that has become resistant to administration of CDK4/6 inhibitors and estrogen antagonists.
Specifically, the invention provides the following [1] to [24].

[1] A therapeutic agent for treating breast cancer that has developed resistance to cyclin-dependent kinase 4/6 (CDK4/6) inhibitors and estrogen receptor antagonists, the therapeutic agent containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof.

[Chemical Formula 2]

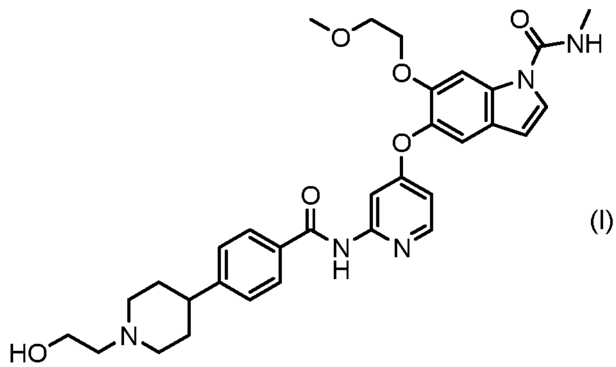

(I)

[2] A therapeutic agent for treating breast cancer, containing a CDK4/6 inhibitor and estrogen receptor antagonist, which is administered in combination with 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof.

[Chemical Formula 3]

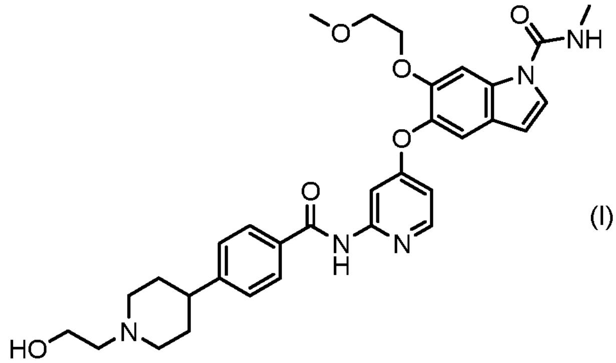

(I)

[3] A therapeutic agent for treating breast cancer, containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, which is administered in combination with a CDK4/6 inhibitor and estrogen receptor antagonist.

[Chemical Formula 4]

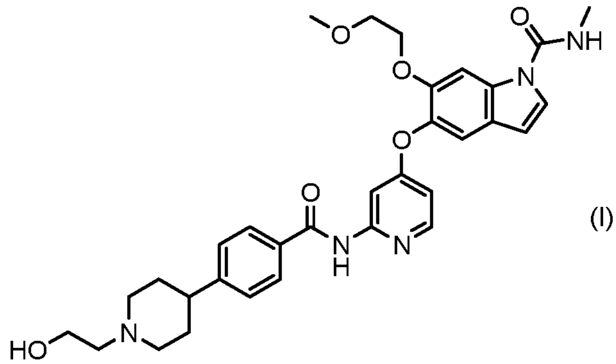

(I)

[4] The therapeutic agent according to [2] or [3] above, wherein the 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or pharmacologically acceptable salt thereof is administered after administration of the CDK4/6 inhibitor and estrogen receptor antagonist.

[Chemical Formula 5]

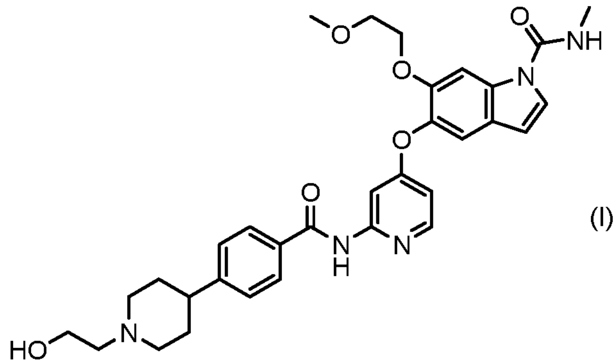

(I)

[5] A composition for treating breast cancer that has developed resistance to CDK4/6 inhibitors and estrogen receptor antagonists, the composition containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable

salt thereof.

[6] A composition for treating breast cancer, containing a CDK4/6 inhibitor and estrogen receptor antagonist, which is administered in combination with 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof.

[7] A composition for treating breast cancer, containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, which is administered in combination with a CDK4/6 inhibitor and estrogen receptor antagonist.

[8] The composition according to [6] or [7] above, wherein the 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or pharmacologically acceptable salt thereof is administered after administration of the CDK4/6 inhibitor and estrogen receptor antagonist.

[9] A method for treating breast cancer that has developed resistance to CDK4/6 inhibitors and estrogen receptor antagonists, wherein the method includes administering 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, to a patient in need of the same.

[10] A method for treating breast cancer, which includes administering 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, a CDK4/6 inhibitor and an estrogen receptor antagonist, to a patient in need of the same.

[11] The method according to [10] above, which includes administering 5-((2-(4-(1-(2-hydroxyethyl)piperidin-4-yl)benzamide)pyridin-4-yl)oxy)-6-(2-methoxyethox y)-N-methyl-1H-indole-1-carboxamiderepresentedbyformula(I) orapharmacologically acceptable salt thereof to a patient in need of the same, after administering the CDK4/6 inhibitor and estrogen receptor antagonist.

[12] The compound 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, for use in treatment of breast cancer that has developed resistance to CDK4/6 inhibitors and estrogen receptor antagonists.

[13] The use of 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, for production of a therapeutic agent for treating breast cancer that has developed resistance to CDK4/6 inhibitors and estrogen receptor antagonists.

[14] The aforementioned therapeutic agent, composition, treatment method, compound or use, wherein the pharmacologically acceptable salt is a 1.5 succinate.

[15] The aforementioned therapeutic agent, composition, treatment method, compound or use, wherein the breast cancer is breast cancer that has developed resistance to CDK4/6 inhibitors and estrogen receptor antagonists.

[16] The aforementioned therapeutic agent, composition, treatment method, compound or use, wherein the breast cancer is estrogen receptor-positive.

[17] The aforementioned therapeutic agent, composition, treatment method, compound or use, wherein the breast cancer is human epidermal growth factor receptor type 2 (HER2)-positive.

[18] The aforementioned therapeutic agent, composition, treatment method, compound or use, wherein the CDK4/6 inhibitor is one or more selected from the group consisting of palbociclib, abemaciclib and ribociclib.

[19] The aforementioned therapeutic agent, composition, treatment method, compound or use, wherein the CDK4/6 inhibitor is palbociclib.

[20] The aforementioned therapeutic agent, composition, treatment method, compound or use, wherein the estrogen receptor antagonist is one or more selected from the group consisting of tamoxifen, fulvestrant and mepitiostane.

[21] The aforementioned therapeutic agent, composition, treatment method, compound or use, wherein the estrogen receptor antagonist is fulvestrant.

[22] The aforementioned therapeutic agent, composition, treatment method, compound or use, wherein the breast cancer is locally advanced breast cancer, metastatic breast cancer, recurrent breast cancer or unresectable breast cancer.

[23] The aforementioned therapeutic agent, composition, treatment method, compound or use, wherein the breast cancer expresses fibroblast growth factor receptor (FGFR).

[24] The aforementioned therapeutic agent, composition, treatment method, compound or use, wherein the FGFR is FGFR1, FGFR2 or FGFR3.

## Advantageous Effects of Invention

The compound represented by formula (I) can potentially exhibit a tumor volume reducing effect for breast cancer that has developed resistance to administration of CDK4/6 inhibitors and estrogen antagonists.

## Brief Description of Drawings

Fig. 1 is a graph showing transition of mean tumor volume of different groups after initiating drug administration.
Fig. 2 is a graph showing transition of mean tumor volume of different groups after initiating drug administration.

## Description of Embodiments

The compound represented by formula (I) and its pharmacologically acceptable salts according to the present invention can be produced by the method described in PTL 1.
As used herein, "pharmacologically acceptable salt" refers to a salt of an inorganic acid, a salt of an organic acid, or a salt of an acidic amino acid, for example.
Preferred examples of salts with inorganic acids include salts with hydrochloric acid, hydrobromic acid, sulfuric acid, nitric acid and phosphoric acid.
Preferred examples of salts with organic acids include salts with acetic acid, succinic acid, fumaric acid, maleic acid, tartaric acid, citric acid, lactic acid, stearic acid, benzoic acid, methanesulfonic acid, ethanesulfonic acid and *p*-toluenesulfonic acid.
Preferred examples of salts with acidic amino acids include salts with aspartic acid and glutamic acid.
Preferred pharmacologically acceptable salts are succinic acid salts or maleic acid salts, with succinic acid salts being more preferred. Particularly preferred are 1.5 succinic acid salts (hereunder, the 1.5 succinic acid salt of the compound represented by formula (I) will be listed as compound A).
The breast cancer therapeutic agent of the invention may be orally administered in the form of a solid formulation such as a tablet, granules, fine granules, powder or capsule, or a liquid drug jelly or syrup. The breast cancer therapeutic agent of the invention may also be administered parenterally in the form of an injection, suppository, ointment or poultice.
The breast cancer therapeutic agent of the invention may be formulated by a method described in the Japanese Pharmacopoeia, 17th Edition.
The dose of the compound represented by formula (I) or its pharmacologically acceptable salt may be appropriately selected according to the severity of symptoms, the age, gender, body weight and sensitivity of the patient, the method of administration, the period of administration, the interval of administration and the type of medical formulation. Normally, it will be 0.5 mg to 5 g, preferably 1 mg to 1 g and more preferably 1 mg to 500 mg per day, for oral administration to adults (60 kg body weight). It may be administered in 1 to 3 dosages per day.
As used herein, a "cyclin-dependent kinase 4/6 (CDK4/6) inhibitor" is a drug that inhibits activity of CDK4 and CDK6, which are enzymes activated in cancer cells and promoting unregulated cell division.
Preferred examples of CDK4/6 inhibitors include palbociclib, abemaciclib and ribociclib. Palbociclib is most preferred among these.
The dosage and method of administration for a CDK4/6 inhibitor, using palbociclib for an ordinary adult as an example, may be oral administration of 125 mg once per day for a continuous period of 3 weeks, followed by a subsequent period of pause for one week This cycle of administration may then be repeated.
When the CDK4/6 inhibitor is ribociclib used for an ordinary adult, it may be administered orally at 600 mg once per day for a continuous period of 3 weeks, followed by a subsequent period of pause for one week This cycle of administration may then be repeated.
When the CDK4/6 inhibitor is abemaciclib used for an ordinary adult, it may be administered orally at 150 mg per dose, given twice per day.
As used herein, a "estrogen receptor antagonist" is a drug that binds to estrogen receptors expressed in breast cancer cells. This mechanism can be used to inhibit binding between estrogen receptors and estrogen, thereby reducing proliferation of breast cancer cells.
Preferred examples of estrogen receptor antagonists include tamoxifen, fulvestrant and mepitiostane, with fulvestrant being most preferred.
The dosage and method of administration of an estrogen receptor antagonist, using fulvestrant as an example, may be intramuscular administration of 500 mg for each dose, at the start, 2 weeks later and 4 weeks later, and every 4 weeks thereafter.
When the estrogen receptor antagonist is tamoxifen, it may be orally administered at 20 to 40 mg per day.
When the estrogen receptor antagonist is mepitiostane, it may be orally administered at 20 mg per day divided into two doses.

An aromatase inhibitor which inhibits estrogen synthesis may also be used instead of an estrogen receptor antagonist. Preferred examples of aromatase inhibitors include anastrozole, letrozole and exemestane.
As used herein, "breast cancer" means a benign or malignant tumor developing in the mammary glands (lactiferous ducts or lobules). Included in this definition are locally advanced breast cancer, metastatic breast cancer, recurrent breast cancer and unresectable breast cancer.
The term "resistance" as used herein means attenuation or loss of drug effect during the course of treatment of a breast cancer patient with a CDK4/6 inhibitor and estrogen receptor antagonist.
As used herein, the phrase "the compound represented by formula (I) or a pharmacologically acceptable salt thereof is administered after administration of a CDK4/6 inhibitor and estrogen receptor antagonist" means that the compound represented by formula (I) or its pharmacologically acceptable salt is administered alone after resistance has developed against administration of the CDK4/6 inhibitor and estrogen receptor antagonist.

## Examples

The present invention will now be explained in greater detail by the following examples.
Example 1 Growth inhibition effect of compound A after palbociclib and fulvestrant administration for human breast cancer patient-derived tumor (OD-BRE-0438) Using NOD-SCID mice (NOD.CB 17-Prkdcscid/J, female, Charles River Japan), with 5 to 6 mice per group, the antitumor effect of administering compound A after completion of an administration period with palbociclib and fulvestrant was evaluated.
OD-BRE-0438 is a tumor line from an estrogen receptor-positive breast cancer patient, established by Oncodesign Co. The tumor slices were grafted under the mouse skin and subcultured. The tumors excised from the mice were cut to about 4 mm-square, and a trocar (φ3.5 mm) was used for grafting under the skin of the right flank of each mouse.
A 1 mg/mL solution of β-estradiol (FujiFilm-Wako) was prepared with 99.5% ethanol (FujiFilm-Wako). It was then adjusted to a final concentration of 2.5 μg/mL using supply-use sterilized water. The mouse were given free access to food from the day of tumor grafting until the end of the test.
The long and short diameters of the tumors were measured using an electronic digital caliper (Digimatic™ caliper by Mitsutoyo Corp.). The tumor volumes were calculated by the following formula.

$$\text{Tumor volume (mm}^3\text{)} = \text{Long diameter (mm)} \times \text{short diameter (mm)} \times \text{short diameter (mm)} / 2$$

Commercially available palbociclib isethionate (Selleck Chemicals) was adjusted to 10 mg/mL concentration with 50 mM sodium lactate buffer (pH 4.0). A commercially available fulvestrant formulation (trade name: Faslodex Intramuscular Injection 250 mg, by AstraZeneca) was used directly.
Palbociclib and fulvestrant administration was initiated from the 26th day after tumor grafting. Palbociclib was orally administered once per day for 14 days at a dosage of 100 mg/kg (10 mL/kg). Fulvestrant was hypodermically injected on the 26th and 33rd day, at a dosage of 250 mg/kg (5 mL/kg).
On the 40th day, the mice were divided into groups in such a manner that the average tumor volumes were approximately the same in each group. The control group consisted of 6 mice, and the compound A-administered group consisted of 5 mice, at different dosages.
Compound A was dissolved in purified water to a concentration of 2.5 mg/mL. Compound A was orally administered to the mice in each group at a dose of 25 mg/kg (10 mL/kg) or 50 mg/kg (20 mL/kg), once per day for 14 days. The control group was untreated.
The tumor volume and body weight of each mouse were measured on day 3, 7, 10 and 14 after starting administration of compound A. The changes in mean tumor volume in each group are shown in Table 1 and Fig. 1.

[Table 1]

| Changes in mean tumor volume after starting administration of compound A | | | | | |
|---|---|---|---|---|---|
| | Day 0 | Day 3 | Day 7 | Day 10 | Day 14 |
| Control group ($mm^3$) | 278.1 | 323.1 | 380.2 | 408.9 | 429.6 |
| Compound A 25 mg/kg group ($mm^3$) | 261.4 | 211.9 | 167.6 | 138.3 | 134.1 |
| Compound A 50 mg/kg group ($mm^3$) | 262.6 | 174.8 | 136.1 | 110.4 | 96.6 |

Reference Example 1 Growth inhibiting effect of compound A on human breast cancer patient derived tumor

(OD-BRE-0438)

The antitumor effect of administering compound A was evaluated using NOD-SCID mice (NOD.CB17-Prkdcscid/J, female, Charles River Japan), with 6 mice per group.

Tumor slices were grafted under the mouse skin and subcultured. The tumors excised from the mice were cut to about 4 mm-square, and a trocar (φ3.5 mm) was used for grafting under the skin of the right flank of each mouse, for evaluation of the antitumor effect.

A 1 mg/mL solution of β-estradiol (FujiFilm-Wako) was prepared with 99.5% ethanol (FujiFilm-Wako). It was then adjusted to a final concentration of 2.5 μg/mL using supply-use sterilized water. The mice were given free access to food from the day of tumor grafting until the end of the test.

Compound A was dissolved in purified water to a concentration of 2.5 mg/mL. On the 32nd day after tumor grafting, the mice were divided into groups in such a manner that the average tumor volumes were approximately the same in each group. The tumor volumes were calculated by the same method as Example 1.

Compound A was orally administered to the mice in each group at a dose of 50 mg/kg (20 mL/kg), once per day for 14 days. The control group was untreated.

The tumor volume and body weight of each mouse were measured on day 3, 7, 10 and 14 after starting administration of compound A. The changes in mean tumor volume in each group are shown in Table 2 and Fig. 2.

[Table 2]

| Changes in mean tumor volume after starting administration of compound A | | | | | |
|---|---|---|---|---|---|
| | Day 0 | Day 3 | Day 7 | Day 10 | Day 14 |
| Control group ($mm^3$) | 1237.2 | 352.4 | 466.2 | 530.4 | 675.1 |
| Compound A 50 mg/kg group ($mm^3$) | 1234.1 | 267.0 | 338.8 | 380.8 | 403.4 |

## Claims

1. A therapeutic agent for treating breast cancer that has developed resistance to cyclin-dependent kinase 4/6 (CDK4/6) inhibitors and estrogen receptor antagonists, the therapeutic agent containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof.

[Chemical Formula 1]

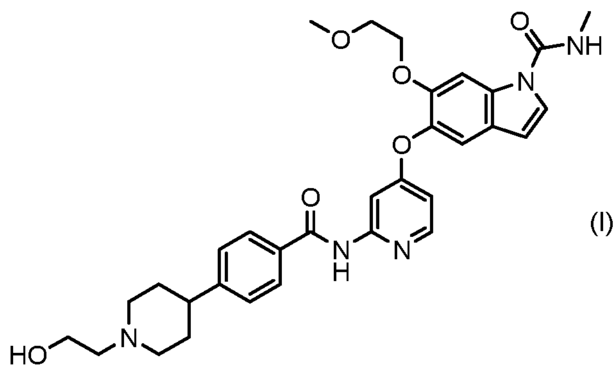

(I)

2. A therapeutic agent for treating breast cancer containing a cyclin-dependent kinase 4/6 (CDK4/6) inhibitor and estrogen receptor antagonist, which is administered in combination with 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof.

[Chemical Formula 2]

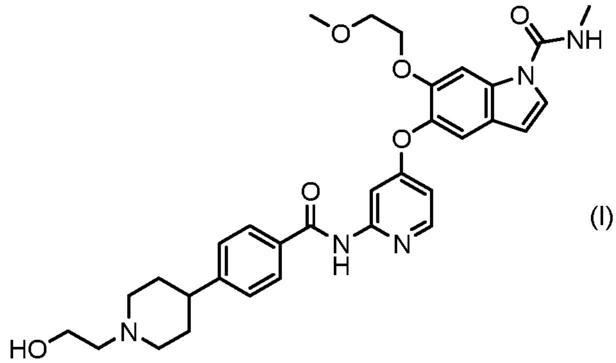

(I)

3. A therapeutic agent for treating breast cancer, containing 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or a pharmacologically acceptable salt thereof, which is administered in combination with a cyclin-dependent kinase 4/6 (CDK4/6) inhibitor and estrogen receptor antagonist.

[Chemical Formula 3]

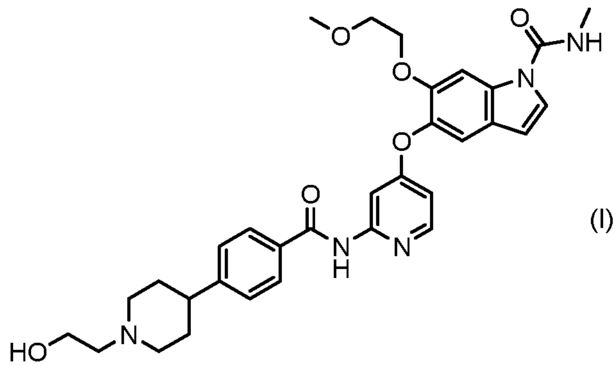

(I)

4. The therapeutic agent according to claim 2 or 3, wherein the 5-((2-(4-(1-(2-hydroxyethyl)piperidin-4-yl)benzamide)pyridin-4-yl)oxy)-6-(2-methoxyethox y)-N-methyl-1H-indole-1-carboxamide represented by formula (I) or pharmacologically acceptable salt thereof is administered after administration of the CDK4/6 inhibitor and estrogen receptor antagonist.

[Chemical Formula 4]

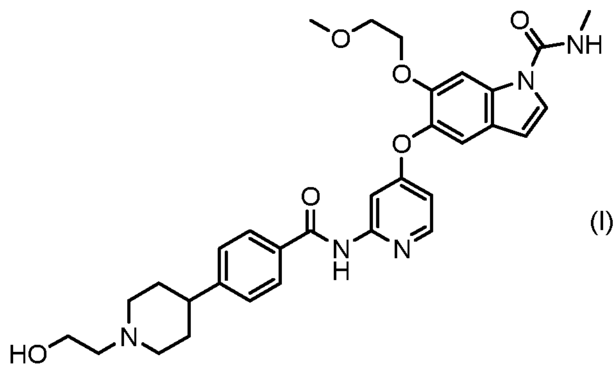

(I)

5. The therapeutic agent according to any one of claims 2 to 4, wherein the breast cancer is breast cancer that has developed resistance to CDK4/6 inhibitors and estrogen receptor antagonists.

**6.** The therapeutic agent according to any one of claims 1 to 5, wherein the pharmacologically acceptable salt is a 1.5 succinate.

**7.** The therapeutic agent according to any one of claims 1 to 6, wherein the breast cancer is estrogen receptor-positive.

**8.** The therapeutic agent according to any one of claims 1 to 7, wherein the breast cancer is human epidermal growth factor receptor type 2 (HER2)-positive.

**9.** The therapeutic agent according to any one of claims 1 to 8, wherein the CDK4/6 inhibitor is one or more selected from the group consisting of palbociclib, abemaciclib and ribociclib.

**10.** The therapeutic agent according to claim 9, wherein the CDK4/6 inhibitor is palbociclib.

**11.** The therapeutic agent according to any one of claims 1 to 10, wherein the estrogen receptor antagonist is one or more selected from the group consisting of tamoxifen, fulvestrant and mepitiostane.

**12.** The therapeutic agent according to claim 11, wherein the estrogen receptor antagonist is fulvestrant.

**13.** The therapeutic agent according to any one of claims 1 to 12, wherein the breast cancer is locally advanced breast cancer, metastatic breast cancer, recurrent breast cancer or unresectable breast cancer.

**14.** The therapeutic agent according to any one of claims 1 to 13, wherein the breast cancer expresses fibroblast growth factor receptor (FGFR).

**15.** The therapeutic agent according to claim 14, wherein the FGFR is FGFR1, FGFR2 or FGFR3.

Fig.1

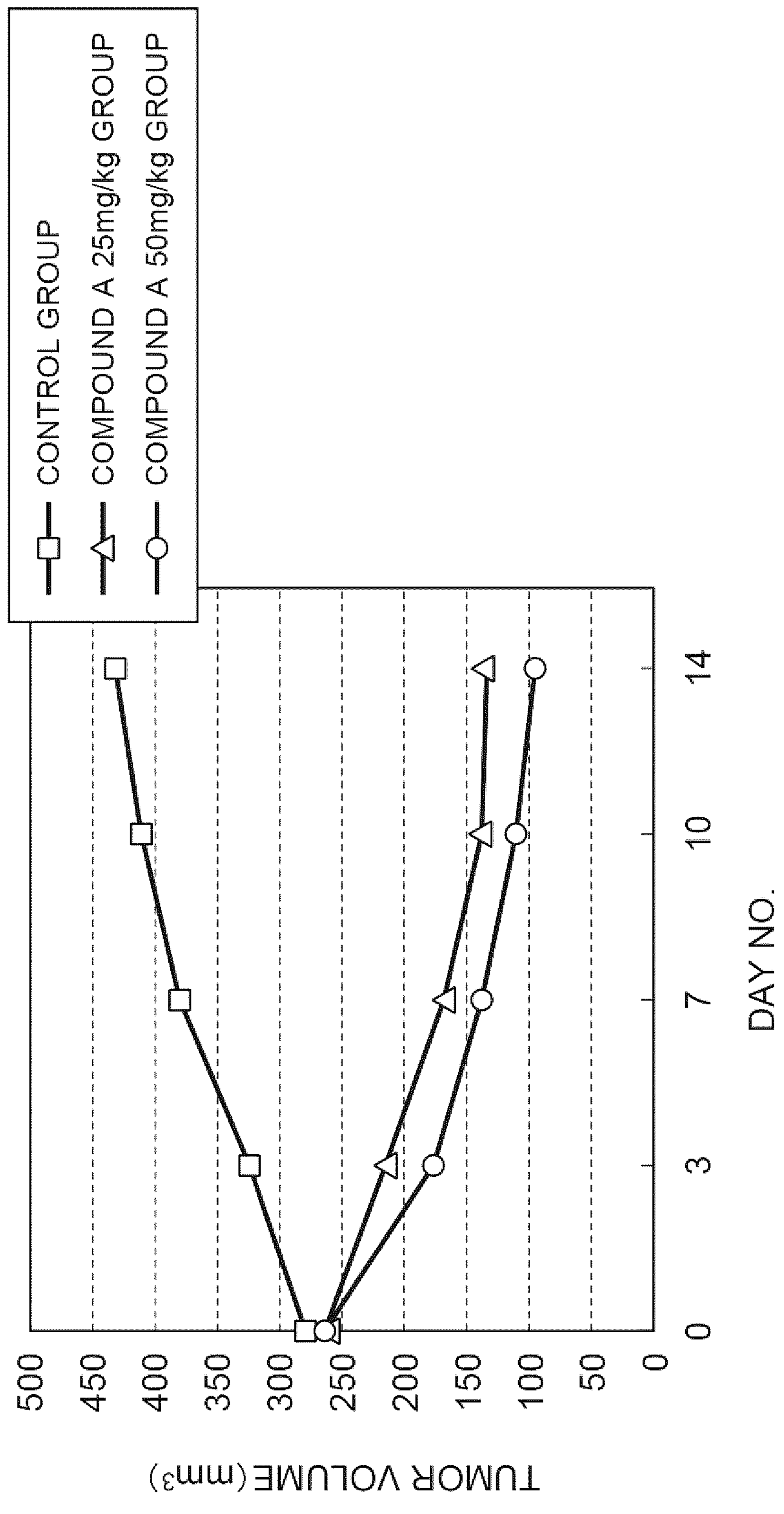
CONTROL GROUP
COMPOUND A 25mg/kg GROUP
COMPOUND A 50mg/kg GROUP
TUMOR VOLUME (mm3)
500
450
400
350
300
250
200
150
100
50
0
0
3
7
10
14
DAY NO.

Fig.2

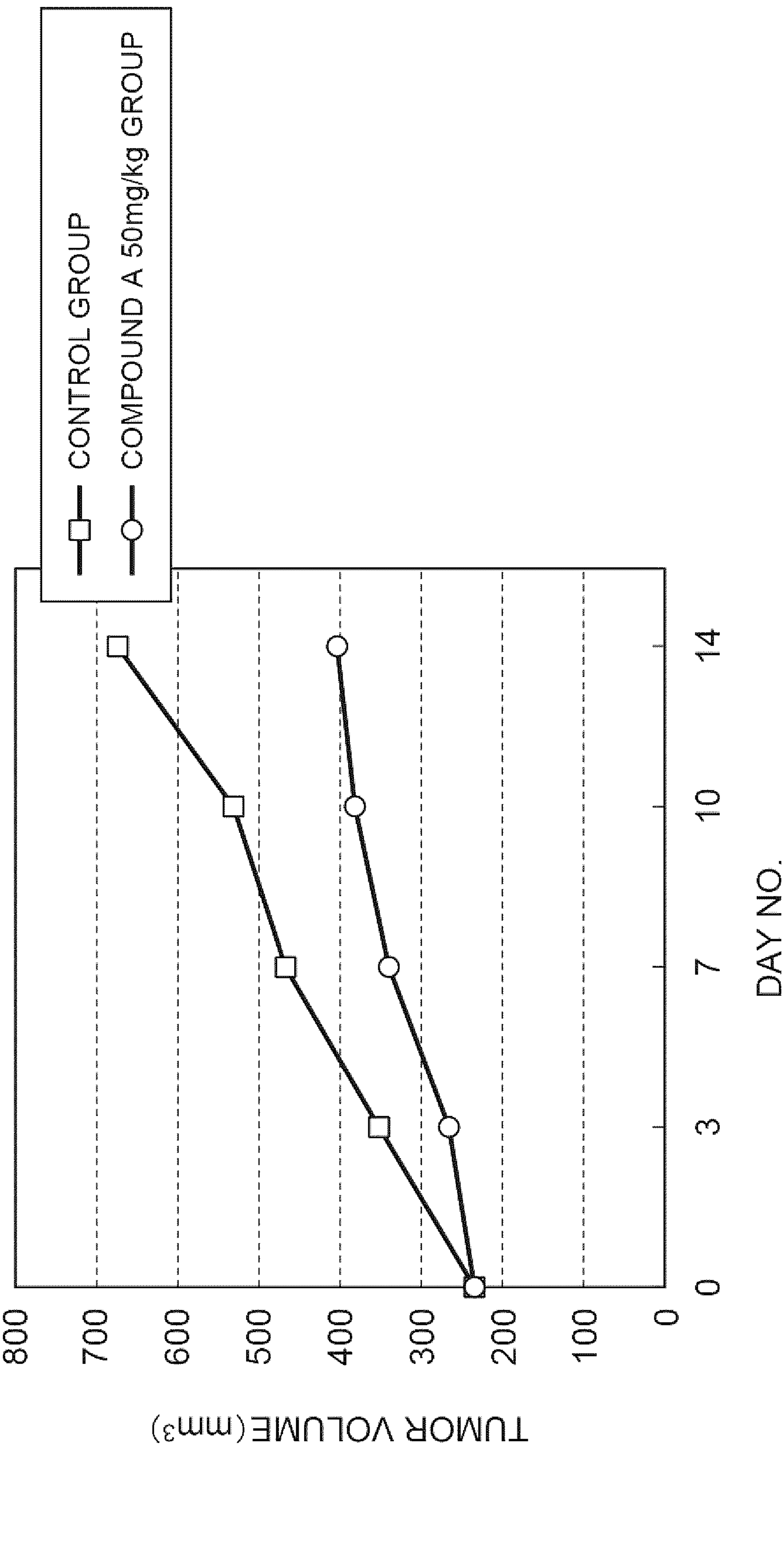
CONTROL GROUP
COMPOUND A 50mg/kg GROUP
TUMOR VOLUME (mm3)
0
100
200
300
400
500
600
700
800
DAY NO.
0
3
7
10
14

## INTERNATIONAL SEARCH REPORT

International application No.
PCT/JP2021/015546

A. CLASSIFICATION OF SUBJECT MATTER
Int. Cl. A61K31/4545(2006.01)i, A61K31/138(2006.01)i, A61K31/519(2006.01)i, A61K31/565(2006.01)i, A61K45/06(2006.01)i, A61P15/00(2006.01)i, A61P35/00(2006.01)i, A61P35/04(2006.01)i, A61P43/00(2006.01)i
FI: A61K31/4545, A61P15/00, A61P35/00, A61P43/00 121, A61K45/06, A61K31/138, A61K31/565, A61K31/519, A61P35/04

According to International Patent Classification (IPC) or to both national classification and IPC

B. FIELDS SEARCHED

Minimum documentation searched (classification system followed by classification symbols)
Int. Cl. A61K31/4545, A61K31/138, A61K31/519, A61K31/565, A61K45/06, A61P15/00, A61P35/00, A61P35/04, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2017/104739 A1 (EISAI R&D MANAGEMENT CO., LTD.) 22 June 2017, claims, paragraphs [0001]-[0003], [0017], [0021] | 1-15 |
| Y | FORMISANO, Luigi et al. Aberrant FGFR signaling mediates resistance to CDK4/6 inhibitors in ER+ breast cancer, nature communications, 2019, vol. 10, 1373, pp. 1-14, https://doi.org/10.1038/s41467-019-09068-2, abstract, p. 2, left column, lines 11-16, p. 7, left column, line 11 to right column, line 10, p. 11, left column, lines 1-6 | 1-15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13.05.2021 | 25.05.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/015546

| Patent Documents referred to in the Report | Publication Date | Patent Family | Publication Date |
|---|---|---|---|
| WO 2017/104739 A1 | 22.06.2017 | US 2018/0303817 A1<br>claims, paragraphs [0001]-[0003], [0019], [0023]<br>CN 108367000 A<br>KR 10-2018-0094862 A<br>EP 3391885 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140235614
- US 20180015079
- US 20180303817
- WO 2019189241 A
- US 20170217935


### Non-patent literature cited in the description


- **BOTTCHER et al.** *Acta Oncologica,* 2019, vol. 58 (2), 147-153
- **HOWELL et al.** *Journal of Clinical Oncology,* 2004, vol. 22 (9), 1605-1613
- **SEKI et al.** *In vivo,* 2019, vol. 33, 2037-2044
- **NAYAR et al.** *Nature Genetics,* 2019, vol. 51, 207-216
- Japanese Pharmacopoeia